Europäisches Patentamt

European Patent Office

Office européen des brevets

(19)

(11) Numéro de publication : **0 206 954 B1**

(12)

# FASCICULE DE BREVET EUROPÉEN

(45) Date de publication du fascicule du brevet :
26.04.89

(51) Int. Cl.⁴ : **C 07 C 67/08, C 07 C 69/63**

(21) Numéro de dépôt : **86420154.6**

(22) Date de dépôt : **13.06.86**

(54) **Procédé de préparation de trifluoroacétate de méthyle.**

(30) Priorité : **14.06.85 FR 8509024**

(43) Date de publication de la demande :
**30.12.86 Bulletin 86/52**

(45) Mention de la délivrance du brevet :
**26.04.89 Bulletin 89/17**

(84) Etats contractants désignés :
**AT BE CH DE FR GB IT LI LU NL SE**

(56) Documents cités :
**Eléments de la technique relevés: néant**

(73) Titulaire : **RHONE-POULENC CHIMIE**
**25, quai Paul Doumer**
**F-92408 Courbevoie Cedex (FR)**

(72) Inventeur : **Amiet, Louis**
**10, quai Saint-Vincent**
**F-69001 Lyon (FR)**

(74) Mandataire : **Le Pennec, Magali et al**
**RHONE-POULENC INTERSERVICES Service Brevets**
**Chimie 25, Quai Paul Doumer**
**F-92408 Courbevoie Cedex (FR)**

## Description

La présente invention concerne un procédé de préparation de trifluoroacétate de méthyle. Ce dernier étant utilisé comme intermédiaire de synthèse dans l'industrie pharmaceutique, une haute pureté est souvent nécessaire. Cette invention permet d'atteindre cet objectif.

Il est décrit dans le « Journal of Organic Chemistry (HAGEN, MILLER, BYNUM, KAPILA, 47, 1345-1347, 1982) » la préparation de trifluoroacétate de méthyle par condensation de l'acide trifluoroacétique avec le méthanol suivie d'une simple distillation.

L'ester obtenu selon cet article avec un rendement de 100 % présente un point d'ébullition de 49-51 °C. Le point d'ébullition de l'ester méthylique de l'acide trifluoracétique pur est en réalité de 41-43 °C. Beilstein (E IV-2 p. 463). Le produit obtenu dans cet article ne peut donc être constitué de trifluoroacétate de méthyle pur.

Il est aussi connu d'après « MOFFAT et HUNT JACS 79, 54 (1957) et 81, 2082-6 (1959) » de préparer les esters de l'acide trifluoracétique par réaction de l'alcool avec un excès d'acide trifluoracétique en présence d'acide sulfurique comme catalyseur suivi d'un lavage à l'eau de l'ester. Lors du lavage à l'eau de l'ester, une hydrolyse partielle se produit, l'excès d'acide trifluoroacétique ainsi que les produits d'hydrolyse sont fortement dilués dans le milieu aqueux à partir duquel ils sont industriellement très difficilement récupérables. Ces deux inconvénients rendent ce procédé inexploitable industriellement, surtout d'un point de vue économique car les matières premières de départ, et particulièrement l'acide trifluoracétique, sont très onéreuses.

Il est également connu d'après le « Research Disclosure N° 229.359 » de condenser l'acide trifluoroacétique avec un alcool aliphatique, contenant une chaîne alkyle courte, en présence d'une quantité catalytique d'acide minéral fort, puis de traiter la phase organique avec un acide minéral fort et enfin de distiller l'ester obtenu. l'acide trifluoracétique et l'alcool sont utilisés en quantités approximativement équimoléculaires.

Il a été constaté dans le cas de la préparation du trifluoroacétate de méthyle, que lorsqu'on employait un léger excès (5 %) de méthanol par rapport à la quantité d'acide trifluoracétique en présence de quantités importantes d'acide sulfurique, le rendement de la réaction d'estérification n'était pas quantitatif et que lorsque l'on employait des quantités catalytiques d'acide sulfurique, on distillait de l'eau avec l'ester ce qui provoquait une légère hydrolyse avec formation d'acide trifluoracétique non récupérable. Si l'on emploie un excès plus important de méthanol, il est impossible de séparer le trifluoroacétate de méthyle pur, à cause de l'existence de l'azéotrope trifluoroacétate de méthyle/méthanol. Si l'on emploie un excès d'acide trifluoracétique par rapport au méthanol, soit on perd l'excès ajouté ce qui n'est pas intéressant d'un point de vue économique, vu le prix de l'acide trifluoracétique, soit on doit élaborer un processus de récupération très compliqué car l'acide trifluoracétique forme lui aussi avec l'eau, un azéotrope.

Ainsi, aucun des procédés de l'art antérieur ne permet de fabriquer le trifluoracétate de méthyle de manière industriellement rentable.

La présente invention a permis d'atteindre cet objectif et a pour objet un procédé de préparation de trifluoroacétate de méthyle caractérisé en ce que dans une première étape, on met en contact l'acide trifluoracétique avec un excès de méthanol et en ce qu'on distille l'azéotrope trifluoroacétate de méthyle/méthanol qui dans une deuxième étape est mis en contact avec de l'acide trifluoracétique en présence de quantité catalytique d'un acide fort et en ce qu'on distille le trifluoroacétate de méthyle obtenu.

Le procédé de la présente invention est un procédé discontinu qui permet d'estérifier tout l'acide trifluoracétique introduit, qui permet d'obtenir un trifluoracétate de méthyle (5) pur et qui évite de soumettre le mélange acide trifluoracétique/eau à une séparation difficile.

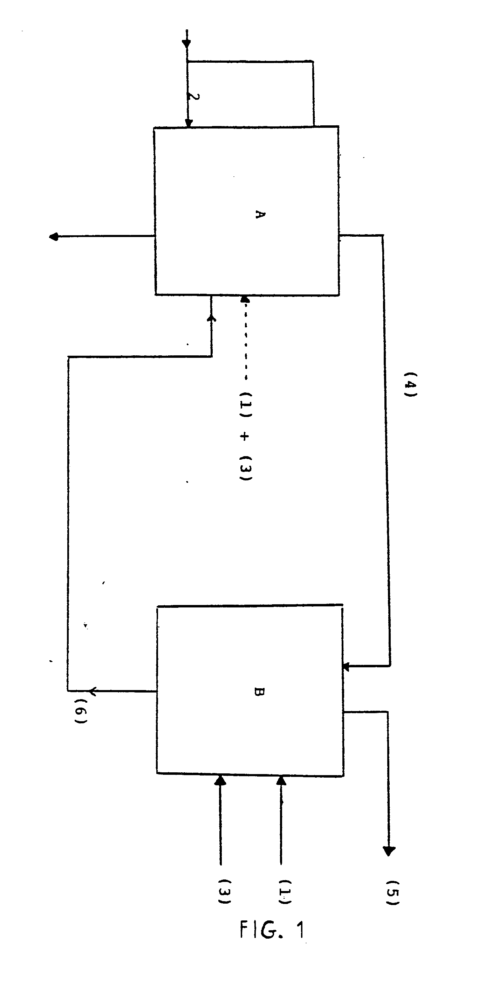

L'invention va être plus complètement décrite à l'aide de la figure 1.

On introduit dans l'enceinte A pour l'initiation de la première étape de l'acide trifluoracétique (1) et du méthanol (2) selon un rapport molaire du méthanol à l'acide trifluoracétique d'au minimum 2,30. On ajoute une quantité catalytique d'un acide fort (3) libre ou supporté, choisi notamment parmi l'acide sulfurique, l'acide phosphorique. On chauffe le réacteur de façon à distiller l'azéotrope trifluoroacétate de méthyle/méthanol (4) dont le point d'ébullition est de 37 °C-38 °C sous une pression de 98000 Pascal et la teneur en méthanol d'environ 6 % en poids. On continue à chauffer le réacteur de façon à distiller le méthanol (1) qui sera ensuite recyclé vers la première étape du cycle suivant. Le résidu du réacteur contenant l'acide minéral, l'eau d'estérification, est éliminé.

Au cours de la deuxième étape, l'azéotrope trifluoroacétate de méthyle/méthanol est introduit dans l'enceinte B, on ajoute un excès d'acide trifluoracétique (1) par rapport au méthanol contenu dans l'azéotrope ainsi qu'une quantité catalytique du même acide minéral fort (3) qu'à la première étape. On distille uniquement le trifluoracétate de méthyle (5) qui a un point d'ébullition situé entre 41 °C et 42 °C sous une pression de 98.000 à 101.000 Pascal. Le résidu (6) de cette deuxième étape contient l'excès d'acide trifluoracétique, l'eau engendrée par l'estérification du méthanol contenu dans l'azéotrope trifluoracétate de méthyle/méthanol ainsi que l'acide minéral fort (3) utilisé comme catalyseur.

Ce résidu (6) est introduit dans l'étape 1 dans les cycles suivants à la place de l'acide trifluoracétique pur et du catalyseur de l'étape 1 mis en oeuvre au cours du 1er cycle dit d'initiation.

Ce procédé permet donc d'introduire de l'acide trifluoracétique uniquement dans le réacteur B, un excès de méthanol uniquement dans le réacteur A qui est recyclé et une quantité catalytique d'acide fort. Les seules pertes sont le catalyseur et l'eau formée lors de l'estérification.

Il est évident pour l'homme de l'art que les réacteurs A et B peuvent être un seul ou deux réacteurs différents, suivant le fait que les deux étapes sont réalisées successivement ou simultanément.

Les acides forts sont choisis de préférence parmi l'acide sulfurique, l'acide phosphorique, libres ou supportés.

Selon une mise en oeuvre préférée de l'invention, le rapport molaire du méthanol introduit dans le réacteur A à l'acide trifluoracétique (1) introduit dans le réacteur B est d'au minimum 2,30 et de préférence, d'au minimum 2,35. En effet, en dessous de cette limite, l'azéotrope trifluoracétate de méthyle/méthanol entraîne également de l'eau.

Le rapport pondéral acide minéral fort à l'acide trifluoroacétique est compris de préférence entre 0,25 et 1 % et encore plus préférentiellement entre 0,3 et 0,5 %.

Le trifluoroacétate de méthyle est utilisé comme intermédiaire de synthèse dans la fabrication de composés à activité phytosanitaire ou pharmaceutique (FR 2 416 883).

l'invention va être plus complètement décrite à l'aide des exemples suivants qui ne doivent pas être considérés comme limitatifs de l'invention.

Exemple 1 Initiation du cycle de préparation

Dans un ballon en verre de 6 litres, muni d'un système d'agitation, d'un thermomètre, d'une ampoule à brome et surmonté d'une colonne à distiller en verre de 30 plateaux (« Oldershaw ») avec système de condensation et de soutirage habituel, on charge successivement 2.201 g d'acide trifluoroacétique pur (dénommé TFA) soit 19,3 moles, puis en 0,5 heure, sous légère agitation, 1.448 g de méthanol pur CH₃OH, enfin 13 g de H₂SO₄ pur concentré (98 %). On porte à reflux sous pression atmosphérique ambiante et la température de tête de colonne se stabilise entre 37,5 et 38 °C. On soutire avec un taux de reflux de 3 à 4, une fraction A de 2.560 g formée d'un mélange, d'après l'analyse, à 91,7 % de TFAM, 1,3 % de TFA et 7 % de CH₃OH. On distille ensuite une fraction B passant à 63 °C formée de CH₃OH, pesant 674,5 g, puis un intermédiaire C de 20 g correspondant au passage de la température de 65 à 95 °C. Il reste un résidu de 320 g, éliminé.

2e étape du cycle

Dans le même appareillage que précédemment, on place 2.064 g de la fraction A précédente, on ajoute 2 271 g de TFA 13 g de H₂SO₄ et porte à reflux. La température se stabilise progressivement à 41-41,5 °C; on soutire alors, avec un reflux de 5, une masse de 2.430 g analysée par RMN comme formée à 99,7 % de TFAM pur. On stoppe la distillation dès l'amorce d'une montée en température à 41,6-41,8 °C et conserve le résidu R.

1e étape du cycle

A la totalité du résidu R, on ajoute 1.490 g de CH₃OH et porte à reflux. Comme pour l'étape d'initiation 1 décrite ci-dessus, on sépare successivement une fraction de 2.087 g passant à 37,5-38 °C contenant 92 % de TFAM, 7 % de CH₃OH et environ 1 % de TFA ; l'excès de méthanol formant 830 g ; un intermédiaire de 18 g. On élimine un résidu pesant 338 g ; son analyse ne permet pas de détecter, dans la limite de sensibilité de la méthode, soit 5 mg/l, d'ion F⁻ et donne une teneur en « F total » de 260 mg/l qui correspond à des traces d'acide trifluoracétique et d'esters.

Exemple 2

On opère dans le même appareillage qu'à l'exemple 1 et selon le même procédé, en mettant en oeuvre, au cours du cycle d'initiation :
2.188 g (19,2 moles) d'acide trifluoracétique
2.000 g (62,5 moles) de méthanol
7 g d'acide sulfurique.
On recueille à 37 °C en tête de colonne, un distillat qui correspond à l'azéotrope trifluoroacétate de méthyle (TFAM)/méthanol de 2.626 g puis 1.188 g de méthanol, une fraction intermédiaire passant de 63 à 97 °C de 22 g et on élimine un résidu de 320 g.

Au cours de la deuxième étape, selon le procédé, on introduit :
2.615 g de l'azéotrope TFAM/méthanol obtenu au cours de l'initiation
2.850 g d'acide trifluoracétique
13,8 g d'acide sulfurique.
On recueille un distillat de trifluoracétate de méthyle de 3.045 g présentant une pureté en TFAM de 99,7 % et il reste un résidu R.

Au cours de la première étape d'un nouveau cycle selon le procédé de l'invention, on introduit dans le réacteur contenant le résidu R :
1.188 g de méthanol venant du distillat de l'étape d'initiation précédente
812 g de méthanol supplémentaire.
On distille :
2.815 g d'un azéotrope TFAM/méthanol puis
1.135 g de méthanol
20 g d'une fraction intermédiaire.
On élimine 435 g de résidu.
Au cours de la deuxième étape de ce nouveau cycle, on introduit
2.790 g d'azéotrope TFAM/méthanol
2.850 g d'acide trifluoracétique
10,5 g d'acide sulfurique concentré à 98 %.
On recueille un distillat de 3.085 g de trifluoracétate de méthyle et un résidu de 2.482 g recyclé éventuellement vers un troisième cycle de préparation.

Exemple 3

Dans un ballon de 0,5 litre muni du même système qu'à l'exemple 1 et d'une colonne à distiller de 10 plateaux, on introduit :

160 g de méthanol (5 moles) puis en 30 minutes sous lente agitation :

175 g d'acide trifluoracétique (1,53 mole).

On ajoute ensuite 1,4 g d'acide phosphorique à 85 %.

Le mélange est chauffé à reflux et la température en tête de colonne se fixe à 38 °C. On distille 195 g d'azéotropetrifluoracétate de méthyle/méthanol.

Au cours de la 2e étape, on introduit :

100 g d'azéotrope TFAM/méthanol obtenu précédemment

114 g d'acide trifluoracétique

1 g d'acide phosphorique.

On porte à reflux et on distille entre 41 et 41,5 °C en tête de colonne, une fraction de 112 g de trifluoracétate de méthyle.

**Revendications**

1. Procédé de préparation de trifluoroacétate de méthyle caractérisé en ce que dans une première étape, on met en contact l'acide trifluoracétique avec un excès de méthanol et en ce qu'on distille l'azéotrope trifluoroacétate de méthyle/méthanol qui dans une deuxième étape est mis en contact avec de l'acide trifluoracétique en présence de quantité catalytique d'un acide fort et en ce qu'on distille le trifluoroacétate de méthyle obtenu.

2. Procédé selon la revendication 1 caractérisé en ce que préalablement à la deuxième étape, on distille l'excès de méthanol qui est recyclé vers la première étape.

3. Procédé selon la revendication 1 caractérisé en ce que le résidu de la deuxième étape après distillation du trifluoroacétate de méthyle est recyclé vers la première étape.

4. Procédé selon la revendication 1 caractérisé en ce que le rapport molaire méthanol à l'acide trifluoroacétique introduit est supérieur ou égal à 2,30 et de préférence supérieur ou égal à 2,35.

5. Procédé selon la revendication 1 caractérisé en ce que le rapport pondéral acide sulfurique à l'acide trifluoracétique est compris entre 0,25 et 1 % et de préférence entre 0,3 et 0,5.

**Claims**

1. Process for the preparation of methyl trifluoroacetate characterized in that, in a first stage, trifluoroacetic acid is brought into contact with an excess of methanol and in that the methyl trifluoroacetate/methanol azeotrope is distilled which, in a second stage, is brought into contact with trifluoroacetic acid in the presence of a catalytic quantity of a strong acid and in that the methyl trifluoroacetate obtained is distilled.

2. Process according to Claim 1 characterized in that, prior to the second stage, the excess methanol is distilled which is recycled towards the first stage.

3. Process according to Claim 1 characterized in that the residue from the second stage after the distillation of methyl trifluoroacetate is recycled towards the first stage.

4. Process according to Claim 1 characterized in that the molar ratio of methanol to the trifluoroacetic acid introduced is greater than or equal to 2.30 and, preferably, greater than or equal to 2.35.

5. Process according to Claim 1 characterized in that the weight ratio of sulphuric acid to trifluoroacetic acid is between 0.25 and 1 % and, preferably, between 0.3 and 0.5.

**Patentansprüche**

1. Verfahren zur Herstellung von Methyltrifluoracetat, dadurch gekennzeichnet, daß man in einem ersten Schritt Trifluoressigsäure mit einem Überschuß an Methanol in Kontakt bringt und das Azeotrop Methyltrifluoracetat/Methanol destilliert, das in einem zweiten Schritt mit Trifluoressigsäure in Gegenwart einer katalytischen Menge einer starken Säure in Kontakt gebracht wird und daß man das erhaltene Methyltrifluoracetat destilliert.

2. Verfahren nach Anspruch 1, dadurch gekennzeichnet, daß man vor dem zweiten Schritt den Überschuß von Methanol abdestilliert, der in den ersten Schritt zurückgeführt wird.

3. Verfahren nach Anspruch 1, dadurch gekennzeichnet, daß der Rückstand des zweiten Schritts nach der Destillation des Methyltrifluoracetats in den ersten Schritt zurückgeführt wird.

4. Verfahren nach Anspruch 1, dadurch gekennzeichnet, daß das molare Verhältnis Methanol zu eingebrachter Trifluoressigsäure größer oder gleich 2,3 und vorzugsweise größer oder gleich 2,35 ist.

5. Verfahren nach Anspruch 1, dadurch gekennzeichnet, daß das Gewichtsverhältnis Schwefelsäure zu Trifluoressigsäure zwischen 0,25 und 1 % und vorzugsweise zwischen 0,3 und 0,5 liegt.

FIG. 1